# EUROPÄISCHE PATENTANMELDUNG

(11) **EP 1 162 186 A2**
(43) Veröffentlichungstag der Anmeldung: **12.12.2001**
(21) Anmeldenummer: 01110082.3
(22) Anmeldetag: 30.04.2001
(51) Int. Cl.: C07C 29/56

(54) **Verfahren zur Herstellung von d,l-Menthol**

(30) Priorität: 12.05.2000 DE 10023283
(71) Anmelder: BAYER AG, 51368 Leverkusen (DE)
(72) Erfinder: Schlemenat, Andreas, Dr., 47800 Krefeld (DE); Langer, Reinhard, Dr., 47918 Tönisvorst (DE); Dreisbach, Claus, Dr., 51065 Köln (DE); Gross, Hans-Jürgen, 47259 Duisburg (DE); Prinz, Thomas, Dr., 51371 Leverkusen (DE); Schulze-Tilling, Andreas, Dr., League City, TX 77573 (US); Friederich, Michael, Dr., 47800 Krefeld (DE); Jentsch, Jörg-Dietrich, Dr., 47799 Krefeld (DE); John, Gerald, Dr., 40547 Düsseldorf (DE)

(57) **Zusammenfassung**

Verfahren zur Herstellung von d,1-Menthol durch katalytische Isomerisierung von Stereoisomeren des Menthols oder Gemischen dieser Isomere bei Temperaturen von 30 bis 170°C in Gegenwart eines Ruthenium-Trägerkatalysators, wobei als Trägermaterial Al₂O₃ eingesetzt wird.

## Beschreibung

Die vorliegende Erfindung betrifft ein neues Verfahren zur Herstellung von d,l-Menthol durch Umlagerung von Stereoisomeren des Menthols an Rutheniumhaltigen Trägerkatalysatoren bei Temperaturen von 30 bis 170°C.

Unter den natürlich vorkommenden cyclischen Terpenalkoholen nimmt das l-Menthol, der Hauptbestandteil des Pfefferminzöls, aufgrund seiner kühlenden und erfrischenden Wirkung eine Sonderstellung ein. l-Menthol findet daher Verwendung als Riech- oder Geschmackstoff und wird in der Arzneimittelindustrie eingesetzt.

Die Mentholherstellung durch katalytische Hydrierung von Verbindungen, die das Kohlenstoffgerüst des Menthans mit wenigstens einer Doppelbindung besitzen und in 3-Stellung durch Sauerstoff substituiert sind (wie z.B. Thymol), führt zu einem Gemisch der acht optisch aktiven Menthole: d,l-Menthol, d,l-Isomenthol, d,l-Neomenthol und d,l-Neoisomenthol. Diese acht optisch aktiven Menthole unterscheiden sich in Bezug auf ihre organoleptischen Eigenschaften. Nur l-Menthol hat den charakteristischen Pfefferminzgeruch und die schon erwähnte erfrischende Wirkung. Es ist deshalb das wertvollste der Menthol-Stereoisomeren. Daher ist man bestrebt, die Hydrierung so zu führen, dass möglichst viel d,l-Menthol entsteht. Durch Auftrennung des Gemisches der acht stereoisomeren Menthole wird dann das d,l-Menthol als Racemat erhalten, das anschließend in die Antipoden gespalten werden kann.

Die Siedepunkte von d,l-Isomenthol (218,6°C bei 760 Torr (1,01 bar); 75-78°C bei 2,5 Torr (3,3 mbar)) und d,l-Menthol (216,5°C bei 760 Torr (1,01 bar); 75-78°C bei 2,5 Torr (3,3 mbar)) liegen sehr nahe beieinander. Die Trennleistung einer Kolonne bei der destillativen Trennung der einzelnen Menthol-Isomere wird daher insbesondere durch das Verhältnis von Menthol zu Isomenthol bestimmt. Für eine hohe Raum-Zeit-Ausbeute an d,l-Menthol bei der destillativen Trennung ist deshalb neben einem möglichst hohen Menthol-Gehalt im zu trennenden Gemisch auch ein möglichst geringer Isomenthol-Gehalt nötig. Die Ausbeute an Menthol wird also bei einer gegebenen Destillationskolonne wesentlich durch das Eingangs-Verhältnis von Menthol zu Isomenthol bestimmt.

Es ist bekannt, zur Herstellung von d,l-Menthol Verbindungen, die das Kohlenstoffgerüst des Menthans mit wenigstens einer Doppelbindung besitzen und in 3-Stellung durch Sauerstoff substituiert sind, beispielsweise Thymol, in kontinuierlichen Prozessen an festen Katalysatorschüttungen mit Wasserstoff zu hydrieren bzw. Stereoisomere des Menthols an festen Katalysatorschüttungen umzulagern.

In der DE 2 314 813 A 1 wird ein Verfahren zur Hydrierung von Verbindungen, die das Kohlenstoffgerüst des Menthans mit wenigstens einer Doppelbindung besitzen und in 3-Stellung durch Sauerstoff substituiert sind, an einer Schüttung eines Kobalt-Mangan-Katalysators bei Temperaturen von 170°C bis 220°C und Drücken über 25 bar, bevorzugt über 200 bar beschrieben. In den Beispielen wird bei Temperaturen von 180°C bis 210°C und bei Drücken über 200 bar gearbeitet und ein Gemisch der acht stereoisomeren Menthole erhalten, das zu 59,5 bis 59,9 % aus dem racemischen d,l-Menthol und zu 10,6 bis 10,8 % aus d,l-Isomenthol besteht. Das Menthol-Isomenthol-Verhältnis beträgt maximal 5,7. Durch Modifikation dieses Katalysators mit Kupfer wurden Menthol-Gemische mit d,l-Menthol-Gehalten von 57,6 % und d,l-Isomenthol-Gehalten von 9,2 % erreicht (Menthol-Isomenthol-Verhältnis 6,3), in denen aber 4 bis 5 % unerwünschte Nebenprodukte in Form von nicht wiederverwertbaren Kohlenwasserstoffen enthalten waren.

Aus der EP 0 563 611 A 1 und der DE 197 18 116 A 1 ist bekannt, dass man die Hydrierung von aromatischen oder teilhydrierten cyclischen Verbindungen, die das Kohlenstoffgerüst des Menthans mit wenigstens einer C=C-Doppelbindung besitzen und in 3-Stellung durch Sauerstoff substituiert sind, mit Wasserstoff an einem Festbettkatalysator durchführen kann, der auf einem Träger, der mit einem Metall der Seltenen Erden (SE) und Mangan dotiert ist, Palladium, Ruthenium oder Rhodium oder ein Gemisch dieser Elemente als Aktivbestandteile und Alkalimetallhydroxide und/oder -sulfate als Promotoren enthält. In den Beispielen wurde bei Temperaturen von 180 bis 240°C und Drücken von 270 bis 300 bar gearbeitet. Es wurden Menthol-Gemische erhalten, die ca. 52 bis 57 % d,l-Menthol enthielten. Dabei wurden 11,5 bis 14,8 % d,l-Isomenthol gebildet (Menthol-Isomenthol-Verhältnis 3,6 bis 4,4).

In der EP 743 296 A 1 werden Katalysatoren beschrieben, die aus trägerfreien, verpressten Pulvern von Kobalt-, Mangan- und Erdalkali-oxiden bzw. -hydroxiden bestehen und bei Temperaturen von 150°C bis 230°C und Drücken von 25 bis 350 bar arbeiten. In den angegebenen Beispielen wird bei Temperaturen über 165°C und bei Drücken von mehr als 200 bar gearbeitet. Auf die Zusammensetzung der entstehenden Menthol-Gemische wird nicht eingegangen.

Die Umlagerung von Stereoisomeren des l-Menthols ist in US-A-5 756 864 beschrieben: Bei Temperaturen von 200 bis 350°C und Wasserstoff-Drücken von 50 bis 350 bar, bevorzugt 100 bis 300 bar wird d-Menthol in einem kontinuierlichen Prozess an einem Katalysator racemisiert und isomerisiert, der aus trägerfreien, gepressten Pulvern von Nickel-, Mangan und Erdalkali-hydroxiden bzw. -oxiden besteht. Dabei wurden Menthol-Gemische erhalten, die maximal zu 59,8 % aus d,l-Menthol bestanden.

Alle bisher beschriebenen Verfahren zur Herstellung von Menthol durch Hydrierung ausgehend von Verbindungen mit dem Kohlenstoffgerüst des Menthans bzw. Isomerisierung von Stereoisomeren des Menthols werden bei hohen Drücken und hohen Temperaturen durchgeführt. In keinem der beschriebenen Verfahren werden mehr als 59,9 % d,l-Menthol gebildet. Der Isomenthol-Gehalt liegt minimal bei etwa 10 bis 11 %.

US-A-2 843 636 beschreibt die Isomerisierung von Stereoisomeren des Menthols zu d,1-Menthol mit Wasserstoff in Gegenwart eines Hydrierkatalysators aus der Gruppe Kupferchromit, Kobalt und Nickel bei 260 bis 280°C und 500 bis 1300 p.s.i.g. (34 bis 90 bar) in Autoklaven. Die entstehenden Gemische wiesen neben ca. 10 bis 12 % d,l-Isomenthol einen d,l-Menthol-Gehalt von 60 bis 64 % auf. Bei dem beschriebenen Niederdruckverfahren fallen jedoch wahrscheinlich aufgrund der sehr hohen Temperatur als Nebenprodukte etwa 5 % nicht wiederverwendbare Kohlenwasserstoffe an.

In der Deutschen Patentanmeldung 198 53 562.7 wird eine Niederdruck-Hydrierung von Thymol an einer stationären Katalysatorschüttung beschrieben, die einen Temperaturgradienten aufweist: Die ersten zwei von fünf in Serie geschalteten Reaktorrohren werden auf 180°C temperiert, die hinteren drei Reaktorrohre auf 80 bis 90°C. Mit einem Katalysator, der auf einem Träger, der mit einem Metall der Seltenen Erden (SE) und mit Mangan dotiert ist, Ruthenium als Aktivbestandteil und Alkalimetallhydroxide als Promotoren enthält, konnte bei einem Druck von 3 bar ein Menthol-Isomerengemisch erhalten werden, das 64,4 % Menthol und 12,1 % Isomenthol enthielt (Menthol/Isomenthol = 5,3). Die Isomerisierung einer wasserstoffgesättigten Mischung aus d,l-Neomenthol, d,l-Isomenthol und d,l-Menthol lieferte bei Normaldruck ein Isomerengemisch mit einer Zusammensetzung von 65,3 % d,l-Menthol und 12,1 % Isomenthol. Bei diesem Niederdruckverfahren lassen sich hohe Menthol-Gehalte von ca. 65 % erzielen. Das Menthol-Isomenthol-Verhältnis beträgt maximal 5,4.

Isomerengemische lassen sich destillativ um so einfacher zu reinem d,l-Menthol aufarbeiten, je höher der Anteil von d,l-Menthol und je höher das Menthol-Isomenthol-Verhältnis ist. Aufgabe der Erfindung war es deshalb, ein selektives und technisch einfaches Verfahren für die Herstellung von d,l-Menthol zu finden, das unter Verwendung eines einfach herzustellenden Katalysators im Produktgemisch d,l-Menthol-Gehalte ≥ 60 % und Menthol-Isomenthol-Verhältnisse von ≥ 6,0 ermöglicht und die Bildung unerwünschter Nebenprodukte möglichst weitgehend vermeidet. Unter den genannten Prozentangaben sind hierbei Flächenprozent zu verstehen, die sich bei der gaschromatographischen Analyse des Produktgemisches ergeben. Unter Menthol-Isomenthol-Verhältnis ist das Verhältnis von Flächenprozent (GC) Menthol zu Flächenprozent (GC) Isomenthol zu verstehen.

Es wurde nun gefunden, dass man ausgehend von den Isomeren des Menthols bzw. von Gemischen dieser Isomeren, die einen d,l-Menthol-Gehalt von 0 bis 60 %, typischerweise von etwa 55 % aufweisen, durch Isomerisierung bei Temperaturen von 30 bis 170°C mit einfachen Ruthenium-Trägerkatalysatoren (Heterogene Katalyse) Menthol-reichere Gemische herstellen kann, die d,l-Menthol-Gehalte von beispielsweise bis zu 67,3 %, Isomenthol-Gehalte von beispielsweise nur 8,2 % und Menthol-Isomenthol-Verhältnisse ≥ 6,0, beispielsweise von bis zu 8,1 aufweisen. Es wurde weiter gefunden, dass die Ruthenium-Trägerkatalysatoren im Betrieb durch Zugabe von Basen regeneriert/reaktiviert werden können.

Die Erfindung ist insofern überraschend, als dass man mit einfach herzustellenden Katalysatoren bei niedrigen Temperaturen und Drücken auch eine Isomerisierung des Isomenthols bewerkstelligen kann. Überraschenderweise wurde gefunden, dass gerade bei geringen Wasserstoffpartialdrücken, insbesondere bei Abwesenheit von Wasserstoff eine sehr hohe Isomerisierungsgeschwindigkeit des Isomenthols beobachtet wird, obwohl die wechselseitige Umlagerung der einzelnen Menthole über eine Dehydrierung/Hydrierung erfolgt.

Gegenstand der Erfindung ist demnach ein Verfahren zur Herstellung von d,l-Menthol durch katalytische Isomerisierung von Stereoisomeren des Menthols oder Gemischen dieser Isomere bei Temperaturen von 30 bis 170°C, wobei die Isomerisierung in Gegenwart eines Ruthenium-Trägerkatalysators durchgeführt und als Trägermaterial Al₂O₃ eingesetzt wird.

Als Edukt können im erfindungsgemäßen Verfahren die einzelnen Isomere des Menthols (Isomenthol, Neomenthol, Neoisomenthol) oder Gemische dieser Isomeren eingesetzt werden, wobei das Edukt bereits Menthol enthalten kann. Es können beispielsweise die Isomerengemische eingesetzt werden, die bei der Racemisierung von optisch aktiven Mentholen anfallen oder die bei der destillativen Abtrennung von d,l-Menthol aus einem Isomerengemisch zurückbleiben. Ferner können beispielsweise die Menthol-Isomerengemische eingesetzt werden, die bei der Hydrierung von cyclischen Verbindungen entstehen, die das Kohlenstoffgerüst des Menthans mit wenigstens einer Doppelbindung besitzen und in 3-Stellung durch Sauerstoff substituiert sind (z.B. Thymol, Menthon, Isomenthon). Dabei kann der Reaktor, in dem die erfindungsgemäße Isomerisierung durchgeführt wird, beispielsweise einem Reaktor zur Hydrierung von cyclischen Verbindungen, die das Kohlenstoffgerüst des Menthans mit wenigstens einer Doppelbindung besitzen und in 3-Stellung durch Sauerstoff substituiert sind, oder einem Reaktor zur Racemisierung/Isomerisierung von d-Menthol oder anderen Isomeren des l-Menthols oder einer z.B. destillativen Trennung eines Gemisches der Isomeren des Menthols nachgeschaltet werden. Der Isomerisierungsreaktor für das erfindungsgemäße Verfahren kann beispielsweise auch zwischen einen bestehenden Hydrierungs-, Isomerisierungs- oder Racemisierungsreaktor und einer folgenden z.B. destillativen Auftrennung geschaltet werden. Gegebenenfalls können verschiedene Produktströme (z.B. aus Hydrierung und Auftrennung) vermischt als Edukt im erfindungsgemäßen Isomerisierungs-verfahren eingesetzt werden. Der Reaktor zur Isomerisierung kann aber gegebenenfalls auch allein betrieben werden.

Das nach dem erfindungsgemäßen Verfahren hergestellte d,l-Menthol-haltige Isomerengemisch kann zur Isolierung von reinem d,l-Menthol aufgetrennt werden beispielsweise durch Destillation.

Das erfindungsgemäße Verfahren kann diskontinuierlich oder kontinuierlich z. B. im Rührkessel, als Rieselphase, in der Sumpfphase mit aufgeschlämmten Katalysator, als Blasensäule oder an einer stationären Katalysatorschüttung durchgeführt werden. Bevorzugt wird das erfindungsgemäße Verfahren in Reaktoren mit stationären Katalysatorschüttungen in der Sumpfphase durchgeführt.

Das erfindungsgemäße Verfahren kann in Gegenwart von Lösungsmitteln durchgeführt werden. Bevorzugt ist jedoch eine lösungsmittelfreie Durchführung.

Die katalytische Isomerisierung kann in dem erfindungsgemäßen Verfahren beispielsweise ohne Zugabe von Wasserstoff im Vakuum (z.B. bei einem Druck von bis herunter zu 50 mbar), bei Normaldruck (1 bar) oder bei erhöhtem Druck bis 300 bar, bevorzugt bei Normaldruck bis 50 bar, ganz besonders bevorzugt bei Normaldruck bis 10 bar durchgeführt werden. Das erfindungsgemäße Verfahren kann auch in Gegenwart von Wasserstoff betrieben werden: Dabei wird die flüssige Phase vor dem Eintritt in den Reaktor mit Wasserstoff gesättigt, ein mit Wasserstoff gesättigter Eduktstrom (z.B. ein Isomerengemisch aus einem Hydrierungsreaktor) eingesetzt oder gasförmiger Wasserstoff zusammen mit dem Edukt in den Reaktor geleitet, so dass ein Wasserstoffpartialdruck zwischen 0,001 bis 300 bar, bevorzugt zwischen 0,01 und 50 bar, besonders bevorzugt zwischen 0,01 und 10 bar eingestellt wird. Bevorzugt wird jedoch bei der erfindungsgemäßen Isomerisierung kein zusätzlicher Wasserstoff eingesetzt, so dass allenfalls der Wasserstoff zugegen ist, der gegebenenfalls über die verwendeten Edukte in den Isomerisierungsreaktor eingetragen wird.

Das erfindungsgemäße Verfahren wird bei Temperaturen von 30 bis 170°C, bevorzugt bei Temperaturen von 50 bis 150°C, besonders bevorzugt bei Temperaturen von 70 bis 140°C durchgeführt.

Für das erfindungsgemäße Verfahren werden Ruthenium-Trägerkatalysatoren (Heterogene Katalyse) eingesetzt. Bevorzugt enthalten die Ruthenium-Trägerkatalysatoren 0,1 bis 15 Gewichtsprozent, besonders bevorzugt 2 bis 9 Gewichtsprozent Ruthenium. Gegebenenfalls werden Ruthenium-Trägerkatalysatoren eingesetzt, die zusätzlich zu Ruthenium 0,1 bis 10 Gewichtsprozent eines oder mehrerer weiterer Metalle der 8. Nebengruppe des Periodensystems (Fe, Co, Ni, Rh, Pd, Os, Ir, Pt) und/oder Sn und/oder Zn, bevorzugt Pt, Sn und/oder Zn enthalten. Die angegebenen Gewichtsprozent sind jeweils auf das Gewicht des Trägermaterials bezogen.

Ausgangsverbindungen zur Herstellung der erfindungsgemäßen Katalysatoren sind daher Verbindungen von Edelmetallen der 8. Nebengruppe des Periodensystems, Zinn oder Zink. Genannt seien beispielsweise die Halogenide, Nitrate, Acetate, organische Komplexe mit Acetylaceton oder Aminosäuren.

Als Trägermaterial wird Al₂O₃ in den verschiedenen Modifikationen, bevorzugt in der γ-Modifikation eingesetzt. Vorteilhaft weist das als Trägermaterial eingesetzte Aluminiumoxid eine BET-Oberfläche von ≥ 100 m²/g, bevorzugt ≥ 160 m²/g, besonders bevorzugt ≥ 180 m²/g auf. Besonders bevorzugt ist Aluminiumoxid, das zusätzlich einem hohen Anteil an makroporösen Poren (> 50 nm) aufweist und ein Porenvolumen von ≥ 300 mm³/g, bevorzugt ≥ 600 mm³/g besitzt. Als geeignete Trägermaterialien seien beispielhaft die kommerziell erhältlichen Al₂O₃-Träger SPH 1515, SPH 531, SPH 501 der Firma Rhodia, D 10-10 der Firma BASF und SA 6176 der Firma Norton genannt.

Der Katalysatorträger kann beispielsweise in Form von Pulver mit Korngrößen von 0,001 bis 0,1 mm, gebrochenem und gesiebtem Material mit Korngrößen zwischen 0,05 bis 5 mm oder in Formkörpern wie Strangpreßlingen, Extrudaten, Pillen, Kugeln oder Granulaten mit Durchmessern von 0,2 bis 30 mm eingesetzt werden.

Zur Herstellung der Katalysatoren kann man beispielsweise so vorgehen, dass man auf eines der genannten Trägermaterialien zunächst Ruthenium und gegebenenfalls eines oder mehrere weitere Metalle der 8. Nebengruppe des Periodensystems und/oder Zinn und/oder Zink aufträgt. Das Auftragen kann durch Behandeln, beispielsweise Tränken oder Besprühen des Trägermaterials mit Lösungen der Metalle geschehen. Dazu werden beispielsweise die Chloride, Acetate und/oder Nitrate eingesetzt. Dieses Aufbringen der Metalle kann in einem Schritt mit gelösten Mischungen der Salze oder nacheinander mit den Lösungen der Einzelverbindungen erfolgen. Nach jedem Auftrag kann der Katalysator getrocknet werden.

Ein in der genannten Weise hergestellter Katalysator wird beispielsweise durch Behandlung mit Wasserstoff oder Wasserstoff-Stickstoffgemischen mit mehr als 1 % Wasserstoffgehalt bei einer Temperatur von 20 bis 400°C, bevorzugt 30 bis 250°C reduziert. Die Reduktion kann auch mit anderen Reduktionsmitteln wie z.B. Hydrazin erfolgen. Der reduzierte Katalysator wird vorteilhaft im Anschluss Chlorid- bzw. Nitrat-frei gewaschen.

Das aufgebrachte Metall kann beispielsweise auch auf dem Träger fixiert werden, indem man den mit Ruthenium und gegebenenfalls weiteren Metallen getränkten Träger mit einer Lösung von basischen Salzen, z.B. Alkali- oder Erdalkalihydroxiden oder -oxiden wie z.B. NaOH, KOH behandelt, wobei das Metall als Oxid oder Hydroxid ausfällt. Ist das Metall auf dem Träger fixiert, können die Reduktion und das Auswaschen löslicher Bestandteile in beliebiger Reihenfolge durchgeführt werden. Bevorzugt werden zunächst die löslichen Bestandteile ausgewaschen und der Katalysator dann reduziert. Die Reduktion kann auch in situ im Reaktor erfolgen, in dem die erfindungsgemäße Isomerisierung durchgeführt werden soll.

Nach jedem Tränkschritt mit Metallsalz-Lösung oder basischen Salzen oder nach Waschungen kann ein Trocknungsschritt durchgeführt werden. Es ist aber auch möglich den Träger ohne Trocknung im nächsten Herstellschritt einzusetzen.

Die Katalysatorbelastung liegt im erfindungsgemäßen Verfahren beispielsweise bei 0,005 bis 5 kg Ausgangsprodukt pro Liter Katalysator und Stunde, bevorzugt bei 0,03 bis 2 kg/l·h, besonders bevorzugt bei 0,06 bis 1,0 kg/l·h. Mit steigender Katalysatorbelastung erhöht sich die Raum-Zeit-Ausbeute der erfindungsgemäßen Isomerisierung. Andererseits nimmt der Anteil an d,l-Menthol im Produktgemisch ab und das Menthol-Isomenthol-Verhältnis sinkt, wobei der Grad der Abnahme stark von der gewählten Reaktionstemperatur abhängt. Die maximale Katalysatorbelastung bei einer gegebenen Reaktionstemperatur, bei der das Produktgemisch ≥ 60 % d,l-Menthol und ein Menthol-Isomenthol-Verhältnis ≥ 6,0 aufweist, ist für den Fachmann leicht bestimmbar.

Das erfindungsgemäße Verfahren führt kaum zur Bildung nicht verwertbarer Nebenprodukte, wie unerwünschte Kohlenwasserstoffe. Die erhaltenen Reaktionsgemische enthalten einen hohen Gehalt an d,l-Menthol, vorzugsweise ≥ 60 %, besonders bevorzugt ≥ 64 %, einen geringen Anteil an d,l-Isomenthol, beispielsweise 8,2 % bis 11 %, bevorzugt 9 % bis 10 % und ein hohes Menthol-Isomenthol-Verhältnis, vorzugsweise 6,0 bis 8,2, besonders bevorzugt 6,5 bis 7,5, ganz besonders bevorzugt 6,8 bis 7,3, so dass die Abtrennung des gewünschten Produktes leicht durchgeführt werden kann, z.B. durch Rektifikation/Destillation.

Der Katalysator weist lange Standzeiten (beispielsweise > 6000 Stunden) auf, in denen eine nur geringe Desaktivierung beobachtet wird. Der Katalysator kann durch Zugabe von geringen Mengen von Basen wie z.B. Alkoholate, Oxide oder Hydroxide der Alkali- oder Erdalkalimetalle (z.B. KOtBu, KOH, NaOH) regeneriert/reaktiviert werden. Die Zugabe von Base kann durch Einwirken einer Lösung einer Base nach Ausbau des Kontaktes oder im Reaktor selbst geschehen. Bevorzugt wird die Base im laufenden Betrieb zum Menthol-Edukt zugegeben, indem eine basische Lösung zum Menthol gegeben wird oder unter Verzicht auf ein Lösungsmittel die Base selbst im Menthol gelöst wird.

### Beispiele

Im Folgenden wird das erfindungsgemäße Verfahren durch Beispiele weiter verdeutlicht, wobei das erfindungsgemäße Verfahren nicht auf die Beispiele beschränkt ist und diese demnach nicht als limitierend zu werten sind. Prozentangaben sind, soweit nicht anders angegeben, Flächenprozent einer gaschromatographischen (GC) Analyse.

### Beispiel 1: Herstellung eines Ru-Trägerkatalysators (6 Gew.-% Ru)

1000 g (ca. 2,24 l) eines handelsüblichen γ-Al₂O₃ mit einer BET-Oberfläche von ca. 255 m²/g (SA 6176 der Fa. Norton, Extrudat mit Teilchendurchmesser 1/16" (ca. 1,6 mm), Schüttdichte ca. 0,44 kg/l) wurde in einem Großrotationsverdampfer (10 l Kolben) vorgelegt, mit einer wässrigen Lösung von 300 g Ruthenium(III)-chlorid (käufliche Lösung mit 20 Gew.-% Ru, 60 g Ru) in 1153 g destilliertem Wasser versetzt und für 10 Minuten rotiert (ca. 16 UpM (Umdrehungen pro Minute)). Das Lösungsmittel wurde bei 90°C und 10 mbar abdestilliert. Der Katalysator wurde im Wasserstoffstrom bei 250°C reduziert. Anschließend wurde der Katalysator solange mit destilliertem Wasser gewaschen, bis das Waschwasser Chlorid-frei war. Danach wurde der Katalysator im Rotationsverdampfer getrocknet (90 °C, 10 mbar).

### Beispiel 2: Versuchsanlage

Die Versuchsanlage besteht aus 5 ölthermostatisierten doppelwandigen Reaktorrohren von jeweils 1 m Länge und 15 mm Innendurchmesser, die übereinander in Reihe verschaltet sind. Hinter jedem Rohrreaktor befindet sich eine Probenentnahmestelle. Die Beheizung der Reaktoren erfolgt durch zwei Thermostate. Die Reaktorrohre wurden jeweils mit ca. 129 ml des Katalysators aus Beispiel 1 gefüllt (Schütthöhe in jedem Rohr ca. 80 cm, Gesamtmenge 643 ml bzw. 284 g). Das eingesetzte Menthol-Isomerengemisch wird mittels einer Membranpumpe in den Rohrreaktor gefördert. Das Edukt kann wahlweise von oben (Rieselphase) oder von unten (Sumpfphase) durch die Rohrreaktore geleitet werden. Die Zugabe von Wasserstoff kann durch Sättigung des Eduktes oder durch Durchleiten von Wasserstoff in einer bestimmten Menge von oben oder unten durch die Reaktorrohre geschehen (Rieselphase, Blasensäule). In der Versuchsanlage sind Betriebsdrücke von Normaldruck bis 30 barÜ (bar Überdruck) möglich. In der Druckfahrweise (Blasensäule, Rieselphase) wird Wasserstoff über einen Druckminderer, der auf den gewünschten Druck eingestellt ist, in die Anlage gefahren. Der Druck in der Anlage wird dadurch konstant gehalten, dass die Abgasmenge über Nadelventile auf eine bestimmte Menge eingestellt (Rotameter) wird. Der Austrag des Produktes erfolgt in einem standgeregelten Produktabscheider (2 Liter). Bei Reaktionsführung ohne Wasserstoff (Sumpfphase, Rieselphase) wird das zum Druckaufbau nötige Gas (beispielsweise Stickstoff) erst direkt vor dem Produktabscheider zugegeben und wieder ausgespeist. Es strömt also nicht durch den Reaktor.

### Beispiel 3: Herstellung von d,l-Menthol

Die Reaktorrohre der Versuchsanlage aus Beispiel 2 wurden auf 100 bis 130°C temperiert. Ein Menthol-Isomerengemisch wurde bei Belastungen zwischen 0,07 und 0,75 kg(Edukt)/l(Katalysator) · h und bei einem Druck von 2 barÜ in einer Sumpfphasen-Reaktion von unten in den Reaktor gepumpt. Wasserstoff wurde nicht zugegeben. Tabelle 1 gibt die Eduktzusammensetzung und die Zusammensetzung des Produktes für die einzelnen Temperaturen und Belastungen an.

**Tabelle 1:**

| Zusammensetzung des Produktes in Abhängigkeit von Temperatur und Belastung; Katalysator 6 Gew.-% Ru auf Al₂O₃, Belastung in kg (Edukt)/l(Katalysator)·h | | | | | | | | |
|---|---|---|---|---|---|---|---|---|
| Temperatur [°C] | Belastung [kg/l*h] | Menthol [%] | Neomenthol [%] | Isomenthol [%] | Neoisomenthol [%] | Menthone (Summe) [%] | Kohlenwasserstoffe [%] | Menthol / Isomenthol |
| Edukt | | 55,8 | 28,5 | 12,2 | 2,20 | 0,34 | 0,99 | 4,57 |
| | | | | | | | | |
| 100 | 0,070 | 65,8 | 23,1 | 9,1 | 0,76 | 0,28 | 0,87 | 7,23 |
| 100 | 0,095 | 65,0 | 23,4 | 9,3 | 0,83 | 0,61 | 0,87 | 7,01 |
| 100 | 0,130 | 65,2 | 23,0 | 9,7 | 0,79 | 0,41 | 0,87 | 6,69 |
| 100 | 0,161 | 65,2 | 22,9 | 10,0 | 0,82 | 0,31 | 0,85 | 6,55 |
| 100 | 0,197 | 64,6 | 22,8 | 10,3 | 0,85 | 0,39 | 1,04 | 6,27 |
| | | | | | | | | |
| 110 | 0,161 | 64,6 | 23,8 | 9,4 | 0,85 | 0,39 | 1,05 | 6,88 |
| 110 | 0,231 | 64,2 | 23,7 | 9,7 | 0,87 | 0,40 | 1,12 | 6,64 |
| 110 | 0,296 | 63,7 | 23,8 | 10,1 | 0,90 | 0,42 | 1,13 | 6,34 |
| | | | | | | | | |
| 120 | 0,299 | 63,2 | 24,5 | 9,7 | 0,99 | 0,64 | 0,91 | 6,52 |
| 120 | 0,419 | 63,1 | 24,5 | 9,9 | 0,99 | 0,53 | 0,95 | 6,34 |
| 120 | 0,592 | 62,4 | 24,7 | 10,3 | 1,03 | 0,58 | 0,97 | 6,03 |
| | | | | | | | | |
| 130 | 0,750 | 61,7 | 25,2 | 10,0 | 1,11 | 0,90 | 1,00 | 6,15 |

### Beispiel 4: Herstellung von d,l-Menthol, Standzeitversuch

Der Versuch aus Beispiel 3 wurde fortgeführt bei 100°C und 2 barÜ mit einer Katalysator-Belastung von 0,067 kg(Edukt)/l(Katalystor)·h. Die Zusammensetzung des entstandenen Isomerengemisches ist in Tabelle 2 angegeben. Nach einer Laufzeit von über 5300 Stunden wurde nur eine geringe Desaktivierung beobachtet.

### Beispiel 5: Herstellung von d,l-Menthol

Der Versuch aus Beispiel 4 wurde fortgeführt mit einem Menthol-Isomerengemisch als Edukt, das ein geringeres Menthol-Isomenthol-Verhältnis aufwies (3,6). Die Eduktzusammensetzung und die Zusammensetzung des Produktes ist in Tabelle 2 angegeben.

### Beispiel 6: Regenerierung des Katalysators

Der Versuch aus Beispiel 5 wurde fortgeführt. Zur Regenerierung des Ruthenium-Trägerkatalysators wurden 2 g Kalium-tert-Butylat in 20 g Methanol gelöst und zum Menthol-Isomerengemisch in die Edukt-Vorlage gegeben (12 l). Die Zugabe von KOtBu wurde einmal wiederholt. Nach Zugabe der Base lieferte der Katalysator vergleichbare Produktzusammensetzungen wie zu Beginn von Beispiel 4 (siehe Tabelle 2).

**Tabelle 2:**

| Produktzusammensetzung der Versuche aus Beispiel 4 bis Beispiel 6 Katalysator 6 Gew.-% Ru auf Al₂O₃, Belastung 0,067 kg (Edukt)/l(Katalysator) · h | | | | | | | | |
|---|---|---|---|---|---|---|---|---|
| | Laufzeit [h] | Menthol [%] | Neomenthol [%] | Isomenthol [%] | Neoisomenthol [%] | Menthone (Summe) [%] | Kohlenwasserstoffe [%] | Menthol/ Isomenthol |
| Beispiel 4 | | | | | | | | |
| | Edukt | 55,8 | 28,5 | 12,2 | 2,20 | 0,34 | 0,99 | 4,57 |
| | | | | | | | | |
| | 1974 | 65,8 | 23,4 | 9,2 | 0,76 | 0,34 | 0,52 | 7,12 |
| | 2866 | 65,7 | 23,3 | 9,5 | 0,77 | 0,31 | 0,40 | 6,90 |
| | 3182 | 65,9 | 23,2 | 9,1 | 0,74 | 0,32 | 0,77 | 7,29 |
| | 3852 | 65,6 | 23,4 | 9,2 | 0,75 | 0,32 | 0,75 | 7,14 |
| | 4404 | 65,6 | 23,3 | 9,3 | 0,75 | 0,31 | 0,72 | 7,08 |
| | 4880 | 65,4 | 23,3 | 9,4 | 0,77 | 0,33 | 0,76 | 6,94 |
| | 5319 | 65,3 | 23,3 | 9,5 | 0,77 | 0,34 | 0,78 | 6,88 |

| Beispiel 5 | | | | | | | | |
|---|---|---|---|---|---|---|---|---|
| | Edukt | 52,1 | 28,4 | 14,4 | 3,23 | 1,22 | 0,74 | 3,62 |
| | | | | | | | | |
| | 5369 | 65,3 | 23,3 | 9,5 | 0,77 | 0,35 | 0,80 | 6,89 |
| | 5621 | 64,5 | 23,6 | 9,4 | 0,77 | 1,17 | 0,58 | 6,85 |

| Beispiel 6 | | | | | | | | |
|---|---|---|---|---|---|---|---|---|
| | Edukt | 56,3 | 27,7 | 12,5 | 2,14 | 0,28 | 1,00 | 4,50 |
| | | | | | | | | |
| | 5767 | 65,2 | 23,3 | 9,6 | 0,77 | 0,34 | 0,80 | 6,78 |
| | | | | | | | | |

| Zugabe von KOtBu | | | | | | | | |
|---|---|---|---|---|---|---|---|---|
| | 6130 | 65,7 | 23,3 | 9,0 | 0,74 | 0,42 | 0,93 | 7,29 |
| | 6772 | 65,6 | 23,2 | 9,2 | 0,75 | 0,40 | 0,85 | 7,15 |
| | 7205 | 65,7 | 23,1 | 9,1 | 0,73 | 0,49 | 0,61 | 7,19 |

### Beispiel 7: Herstellung eines Ru-Trägerkatalysators (4 Gew.-% Ru)

2500 g eines handelsüblichen γ-Al₂O₃ mit einer BET-Oberfläche von ca. 320 m²/g (SPH 501 der Fa. Rhodia, Kugeln, Ø 1,4-2,8 mm, Schüttdichte ca. 0,87 kg/l) wurden wie in Beispiel 1 beschrieben mit einer Lösung von 500 g Ruthenium(III)-chlorid (käufliche Lösung mit 20 Gew.-% Ru, 100 g Ru) in 1100 g destilliertem Wasser behandelt. Nach Trocknung wurde der Kontakt im Wasserstoffstrom bei 250°C reduziert und Chlorid-frei gewaschen.

### Beispiel 8: Herstellung von d,l-Menthol

1964 g (ca. 2250 ml) des Katalysators aus Beispiel 7 wurden in einen doppelwandigen Glasrohrreaktor (Länge 2,05 m, Volumen 2,4 Liter) gefüllt. Die Beheizung des Rohrreaktors erfolgte durch einen Ölthermostaten. Ein Menthol-Isomerengemisch wurde von unten bei Normaldruck bei Temperaturen von 90 bis 120°C ohne Zugabe von Wasserstoff durch den Reaktor geleitet (Sumpfphase). Tabelle 3 gibt Zusammensetzung von Edukt und Produkt für verschiedene Temperaturen und Belastungen an.

**Tabelle 3:**

| Zusammensetzung des Produktes in Abhängigkeit von Temperatur und Belastung, Katalysator 4 Gew.-% Ru auf Al₂O₃, Belastung in kg (Edukt)/l(Katalysator)·h | | | | | | | | |
|---|---|---|---|---|---|---|---|---|
| Temperatur [°C] | Belastung [kg/l*h] | Menthol [%] | Neomenthol [%] | Isomenthol [%] | Neoisomenthol [%] | Menthone (Summe) [%] | Kohlenwasserstoffe [%] | Menthol/ Isomenthol |
| Edukt | | 55,1 | 29,2 | 12,08 | 2,28 | 0,39 | 0,93 | 4,56 |
| | | | | | | | | |
| 90 | 0,012 | 67,3 | 22,5 | 8,52 | 0,63 | 0,43 | 0,64 | 7,90 |
| 90 | 0,025 | 67,0 | 22,4 | 8,76 | 0,63 | 0,43 | 0,79 | 7,66 |
| 90 | 0,031 | 66,6 | 22,5 | 8,95 | 0,64 | 0,43 | 0,80 | 7,44 |
| 90 | 0,056 | 64,0 | 23,8 | 10,16 | 0,70 | 0,42 | 0,94 | 6,30 |
| | | | | | | | | |
| 100 | 0,027 | 66,1 | 22,7 | 8,80 | 0,73 | 0,50 | 1,12 | 7,52 |
| 100 | 0,044 | 65,8 | 23,0 | 8,97 | 0,73 | 0,50 | 0,96 | 7,33 |
| 100 | 0,055 | 65,4 | 23,3 | 9,10 | 0,74 | 0,54 | 0,94 | 7,19 |
| 100 | 0,077 | 64,8 | 23,6 | 9,42 | 0,75 | 0,53 | 0,89 | 6,89 |
| 100 | 0,090 | 64,5 | 23,6 | 9,56 | 0,76 | 0,56 | 0,93 | 6,75 |
| 100 | 0,13 | 63,0 | 24,5 | 10,20 | 0,79 | 0,59 | 0,93 | 6,18 |
| | | | | | | | | |
| 110 | 0,10 | 64,0 | 24,1 | 9,31 | 0,86 | 0,83 | 0,94 | 6,87 |
| 110 | 0,14 | 63,6 | 24,3 | 9,54 | 0,87 | 0,82 | 0,87 | 6,66 |
| 110 | 0,19 | 63,1 | 24,4 | 9,63 | 0,87 | 0,82 | 1,15 | 6,55 |
| | | | | | | | | |
| 120 | 0,19 | 62,4 | 24,5 | 9,51 | 0,98 | 1,42 | 1,13 | 6,57 |
| 120 | 0,24 | 62,2 | 24,7 | 9,62 | 0,98 | 1,40 | 1,12 | 6,47 |
| 120 | 0,32 | 61,5 | 25,2 | 9,94 | 0,99 | 1,38 | 1,01 | 6,19 |

### Beispiel 9: Herstellung eines Ru-Katalysators (6 Gew.-% Ru)

2617,2 g eines handelsüblichen γ-Al₂O₃ mit einer BET-Oberfläche von ca. 255 m²/g (SA 6176 der Fa. Norton, Extrudat mit Teilchendurchmesser 1/16" (1,6 mm), Schüttdichte ca. 0,44 kg/l) wurden wie in Beispiel 1 beschrieben mit einer Lösung von 785,16 g Ruthenium(III)-chlorid (käufliche Lösung mit 20 Gew.-% Ru, 157,0 g Ru) in 2329,2 g destilliertem Wasser behandelt. Nach der Trocknung wurde zur Fixierung des Rutheniums in einem zweiten Tränkschritt eine Lösung von 559 g NaOH in 2555 g destilliertem Wasser aufgebracht, der Katalysator getrocknet, Chlorid-frei gewaschen und erneut getrocknet.

### Beispiel 10: Herstellung von d,l-Menthol

5,81 l (ca. 2,6 kg) des Katalysators aus Beispiel 9 wurden in einen doppelwandigen Rohrreaktor (Volumen 7,4 l; Länge 2,8 m, Ø 5,8 cm) gefüllt, der bei Drücken zwischen Normaldruck und 10 bar betrieben werden kann. Die Beheizung des Rohrreaktors erfolgte durch einen Ölthermostaten. Das eingesetzte Edukt konnte wahlweise von oben (Rieselphase) oder von unten (Sumpfphase) durch den Rohrreaktor geleitet werden. Wasserstoff konnte von unten (Blasensäule) oder von oben (Rieselphase) zusätzlich in den Reaktor geleitet werden. Das Edukt wurde mittels einer Membranpumpe in den Rohrreaktor gefördert. Der Austrag aus dem Rohrreaktor erfolgte in einem standgeregelten Produktabscheider (2 Liter).

Zur Reduktion des Katalysators wurde der Reaktor auf 150°C aufgeheizt und zunächst Formiergas (20 Vol.-% H₂ in N₂), dann reiner Wasserstoff durch den Reaktor geleitet. Der Reaktor wurde auf 100°C abgekühlt und ein Menthol-Isomerengemisch wurde von unten bei Normaldruck ohne Zugabe von Wasserstoff durch den Reaktor geleitet (Sumpfphase, Belastung 0,09 kg(Edukt)/l(Katalysator)·h). Tabelle 4 gibt die Zusammensetzung von Edukt und Produkt an.

Tabelle 4 gibt auch die Produkt-Zusammensetzung bei Zugabe von Wasserstoff, bei Durchführung der Reaktion unter Druck (6 bar) und niedrigeren Temperaturen an.

**Tabelle 4:**

| Zusammensetzung des Produktes in Abhängigkeit von Temperatur, Druck, Belastung und Zugabe von Wasserstoff; Katalysator 6 Gew.-% Ru auf Al₂O₃, Belastung in kg (Edukt)/l(Katalysator) · h | | | | | | | | |
|---|---|---|---|---|---|---|---|---|
| | Laufzeit [h] | Menthol [%] | Neomenthol [%] | Isomenthol [%] | Neoisomenthol [%] | Menthone (Summe) [%] | Kohlenwasserstoffe [%] | Menthol / Isomenthol |
| | Edukt | 56,19 | 27,79 | 12,56 | 2,16 | 0,28 | 1,02 | 4,47 |

| 100°C, Belastung 0,09 kg/l*h, kein Wasserstoff, Normaldruck | | | | | | | | |
|---|---|---|---|---|---|---|---|---|
| | 37 | 64,92 | 23,27 | 8,83 | 0,75 | 0,97 | 1,26 | 7,35 |
| | 157 | 65,12 | 23,32 | 8,86 | 0,75 | 0,81 | 1,13 | 7,35 |
| | 781 | 65,34 | 23,24 | 8,77 | 0,75 | 1,24 | 0,66 | 7,45 |

| 100°C, Belastung 0.10 kg/l*h, 55 l/h Wasserstoff durch den Reaktor (Blasensäule), Normaldruck | | | | | | | | |
|---|---|---|---|---|---|---|---|---|
| | 1094 | 65,04 | 23,25 | 9,26 | 0,77 | 0,80 | 0,88 | 7,03 |

| 100°C, Belastung 0,10 kg/l*h, kein Wasserstoff, Normaldruck | | | | | | | | |
|---|---|---|---|---|---|---|---|---|
| | 1477 | 65,05 | 23,33 | 8,87 | 0,77 | 1,08 | 0,90 | 7,34 |

| 100°C, Belastung 0,11 kg/l*h, kein Wasserstoff (Sumpfphase), 6 barÜ | | | | | | | | |
|---|---|---|---|---|---|---|---|---|
| | 1502 | 65,13 | 23,71 | 9,00 | 0,90 | 0,36 | 0,90 | 7,24 |
| | 1622 | 65,27 | 23,62 | 9,06 | 0,90 | 0,28 | 0,88 | 7,20 |

| 90°C, Belastung 0,03 kg/l*h, kein Wasserstoff (Sumpfphase), 6 barÜ | | | | | | | | |
|---|---|---|---|---|---|---|---|---|
| | 3016 | 66,77 | 22,38 | 8,64 | 0,60 | 0,93 | 0,49 | 7,73 |

| 85°C, Belastung 0,01 kg/l*h, kein Wasserstoff (Sumpfphase), 6 barÜ | | | | | | | | |
|---|---|---|---|---|---|---|---|---|
| | 3170 | 66,81 | 21,73 | 8,20 | 0,62 | 1,80 | 0,59 | 8,15 |
| | 3182 | 66,67 | 21,90 | 8,24 | 0,60 | 1,75 | 0,58 | 8,09 |

### Beispiel 11: Herstellung von d,l-Menthol

2,24 l (ca. 0,93 kg) des Katalysators aus Beispiel 9 wurden in den in Beispiel 8 beschriebenen Reaktor gefüllt und bei Raumtemperatur zunächst eine halbe Stunde lang mit Formiergas (10 Vol.-% H₂ in N₂), dann 5 Stunden mit reinem Wasserstoff reduziert (ca. 60 l/h). Der Reaktor wurde auf 100°C aufgeheizt und ein Menthol-Isomerengemisch von unten bei Normaldruck durch den Reaktor geleitet (Sumpfphase, 100°C). Tabelle 5 gibt die Zusammensetzung von Edukt und Produkt an.

**Tabelle 5:**

| Zusammensetzung des Produktes in Abhängigkeit der Belastung; Katalysator 6 Gew.-% Ru auf Al₂O₃, Belastung in kg (Edukt)/l(Katalysator) · h | | | | | | | | |
|---|---|---|---|---|---|---|---|---|
| | Belastung [kg/l*h] | Menthol [%] | Neomenthol [%] | Isomenthol [%] | Neoisomenthol [%] | Menthone (Summe) [%] | Kohlenwasserstoffe [%] | Menthol / Isomenthol |
| | Edukt | 55,53 | 29,30 | 11,99 | 2,20 | 0,18 | 0,57 | 4,63 |

| 100°C, Kein Wasserstoff | | | | | | | | |
|---|---|---|---|---|---|---|---|---|
| | 0,10 | 64,72 | 23,06 | 8,78 | 0,73 | 1,72 | 0,62 | 7,37 |

| 100°C, Wasserstoff-Sättigung Edukt | | | | | | | | |
|---|---|---|---|---|---|---|---|---|
| | 0,10 | 65,78 | 23,26 | 8,89 | 0,73 | 0,60 | 0,50 | 7,40 |

| 100°C, kein Wasserstoff | | | | | | | | |
|---|---|---|---|---|---|---|---|---|
| | 0,11 | 65,56 | 23,20 | 8,98 | 0,74 | 0,67 | 0,59 | 7,30 |
| | 0,13 | 65,39 | 23,19 | 9,17 | 0,76 | 0,67 | 0,59 | 7,13 |

### Beispiel 12: Herstellung eines Ru-Katalysators (6 Gew.-% Ru)

800 g eines handelsüblichen γ-Al₂O₃ mit einer BET-Oberfläche von ca. 255 m²/g (SA 6176 der Fa. Norton, Extrudat mit Teilchendurchmesser 1/16" (1,6 mm), Schüttdichte ca. 0,44 kg/l) wurden mit einer Lösung von 240 g Ruthenium(III)-chlorid (käufliche Lösung mit 20 Gew.-% Ru, 48 g Ru) in 650 g destilliertem Wasser behandelt. Zu dieser Mischung wurde Natronlauge zugegeben (683,5 g NaOH in 2964 g Wasser) und 20 Stunden bei Raumtemperatur stehengelassen. Der Katalysator wurde abfiltriert, mit Wasser Chlorid-frei gewaschen und bei 90°C im Vakkum getrocknet.

### Beispiel 13: Herstellung von d,l-Menthol

682 g des Katalysators aus Beispiel 12 wurden in den in Beispiel 8 beschriebenen Reaktor gefüllt und bei Raumtemperatur zunächst eine halbe Stunde lang mit Formiergas (10 Vol.-% H₂ in N₂), dann 5 Stunden mit reinem Wasserstoff reduziert (ca. 60 l/h). Der Reaktor wurde auf 100°C aufgeheizt und ein Menthol-Isomerengemisch von unten bei Normaldruck durch den Reaktor geleitet (Sumpfphase). Tabelle 6 gibt die Zusammensetzung von Edukt und Produkt an.

**Tabelle 6:**

| Produktzusammensetzung der Versuche aus Beispiel 13; Katalysator 6 Gew.-% Ru auf Al₂O₃, 100 °C, Belastung 0,20 kg (Edukt)/l(Katalysator)·h, keine Wasserstoffzugabe | | | | | | | |
|---|---|---|---|---|---|---|---|
| Laufzeit [h] | Menthol [%] | Neomenthol [%] | Isomenthol [%] | Neoisomenthol [%] | Menthone [%] | Kohlenwasserstoffe [%] | Menthol/ Isomethol |
| Edukt | 55,8 | 29,2 | 12,0 | 2,20 | 0,17 | 0,44 | 4,65 |
| | | | | | | | |
| 519 | 65,8 | 23,1 | 9,25 | 0,75 | 0,45 | 0,45 | 7,11 |
| 1458 | 65,5 | 23,1 | 9,29 | 0,75 | 0,48 | 0,49 | 7,05 |

## Patentansprüche

1. Verfahren zur Herstellung von d,l-Menthol durch katalytische Isomerisierung von Stereoisomeren des Menthols oder Gemischen dieser Isomere bei Temperaturen von 30 bis 170°C, **dadurch gekennzeichnet, dass** die Isomerisierung in Gegenwart eines Ruthenium-Trägerkatalysators durchgeführt wird, wobei als Trägermaterial Al₂O₃ eingesetzt wird.

2. Verfahren nach Anspruch 1, **dadurch gekennzeichnet, dass** der Ruthenium-Trägerkatalysator 0,1 bis 15 Gewichtsprozent Ruthenium bezogen auf das Gewicht des Trägers enthält.

3. Verfahren nach mindestens einem der Ansprüche 1 und 2, **dadurch gekennzeichnet, dass** der Ruthenium-Trägerkatalysator neben Ruthenium 0,1 bis 10 Gewichtsprozent bezogen auf das Gewicht des Trägers eines oder mehrerer weiterer Metalle der 8. Nebengruppe des Periodensystems und/oder Zinn und/oder Zink enthält.

4. Verfahren nach mindestens einem der Ansprüche 1 bis 3, **dadurch gekennzeichnet, dass** als Trägermaterial Aluminiumoxid in der γ-Modifikation eingesetzt wird.

5. Verfahren nach mindestens einem der Ansprüche 1 bis 4, **dadurch gekennzeichnet, dass** das als Trägermaterial eingesetzte Aluminiumoxid eine BET-Oberfläche von ≥ 100 m²/g, bevorzugt ≥ 160 m²/g, besonders bevorzugt ≥ 180 m²/g aufweist.

6. Verfahren nach mindestens einem der Ansprüche 1 bis 5, **dadurch gekennzeichnet, dass** als Trägermaterial makroporöses Aluminiumoxid mit einem Porenvolumen von ≥ 300 mm³/g, bevorzugt ≥ 600 mm³/g eingesetzt wird.

7. Verfahren nach mindestens einem der Ansprüche 1 bis 6, **dadurch gekennzeichnet, dass** die Isomerisierung bei einem Druck von 50 mbar bis 300 bar durchgeführt wird.

8. Verfahren nach mindestens einem der Ansprüche 1 bis 7, **dadurch gekennzeichnet, dass** bei der Isomerisierung kein zusätzlicher Wasserstoff zugesetzt wird.

9. Verfahren nach mindestens einem der Ansprüche 1 bis 7, **dadurch gekennzeichnet, dass** bei der Isomerisierung Wasserstoff mit einem Partialdruck von 0,001 bis 300 bar zugesetzt wird.

10. Verfahren nach mindestens einem der Ansprüche 1 bis 9, **dadurch gekennzeichnet, dass** das erhaltene Produkt einen d,l-Menthol-Gehalt von mindestens 60 % und ein Menthol-Isomenthol-Verhältnis von mindestens 6,0 aufweist.

11. Verfahren nach mindestens einem der Ansprüche 1 bis 10, **dadurch gekennzeichnet, dass** der Ruthenium-Trägerkatalysator durch Zugabe einer Base regeneriert wird.

12. Verwendung des gemäß Anspruch 1 erhaltenen d,l-Menthol-haltigen Isomerengemischs zur Isolierung von reinem d,l-Menthol.
